# EUROPEAN PATENT APPLICATION

(11) **EP 1 227 106 A1**
(43) Date of publication of application: **31.07.2002**
(21) Application number: 01101800.9
(22) Date of filing: 26.01.2001
(51) Int. Cl.: C07K 14/47, C12N 15/12

(54) **Pro-apoptotic proteins and DNA molecules encoding them**

(71) Applicant: Boehringer Ingelheim International GmbH, 55216 Ingelheim (DE)
(72) Inventor: Hofmann, Johannes, 1180 Wien (AT); Beug, Hartmut, 1140 Wien (AT)
(74) Representative: Laudien, Dieter, Dr.

(57) **Abstract**

The invention provides a novel proapoptotic inducer protein "p12", that was identified in myeloid/erythroid cytokine dependent cells, and DNA molecules encoding it. The p12 protein and DNA molecules are useful for therapy and diagnosis of conditions which are associated with a deregulated apoptosis pathway and for screening compounds that modulate p12 activity. In presence of IL-3 p12 was found in the cytosol, whereas after cytokine removal the cellular p12-level increase and now the majority of cellular p12 was found in the mitochondria fraction. Immunoprecipitation assays suggested that p12 plays an important role in the voltage dependent anion channel (VDAC) dependent release of proapoptotic activities in cytokine dependent myeloid cells.

## Description

The present invention relates to the therapy and diagnosis of disorders associated with a deregulation of molecular pathways involved in apoptosis.

The survival of individual cells in multicellular organisms depends on extracellular signals that are provided by growth factors, cytokines, hormones and extracellular matrix. Furthermore, lineage specificity of survival factors and matrix components can serve to prevent cells from accumulating in ectopic positions (reviewed in Raff, 1996). Withdrawal of growth factors or removal from matrix contact triggers programmed cell death. Aberrant resistance to apoptosis participates in the development of neoplasia, whereas excessive cell death through apoptosis or necrosis contributes to acute organ failure as well as to chronic diseases. Diverse sets of signal transduction pathways have been associated with receptors that promote cell survival. It is therefore not clear what these pathways have in common to promote cell survival (Jacobson et al., 1997). While the steps leading to apoptosis have been partially clarified in some instances (e.g. in death receptor signalling or in the function of certain oncogenes), comparatively little is known how withdrawal of cytokines leads to apoptosis of hematopoietic cells.

On the other hand, the molecular mechanisms of the apoptotic execution program have been conserved throughout evolution and many of the key regulators and executioners have been identified. Widely different pathways are known that activate this machinery and thus commit cells to apoptosis. These pathways involve complex interactions of pro-apoptotic and anti-apoptotic regulators. They include Bcl-2 family members (Chao and Korsmeyer, 1998), caspase family members (Nicholson and Thornberry, 1997, Raff, 1998) and their adaptors like Apaf-1 and Fadd. Caspases are synthesized as dormant proenzymes that, upon proteolytic activation involving interaction with their specific adaptors, acquire the ability to cleave key intracellular substrates. This results in the morphological and biochemical changes associated with apoptosis. Recently it was found that in apoptosis triggered by many stimuli, mitochondria play a pivotal role in coordinating caspase activation through the release of proapoptotic activities (e.g.: cytochrome c, AIF, Diablo/Smac). Release of proapoptotic activities from the mitochondria is controlled by proteins of the Bcl-2 family: those that prevent cell death (Bcl-2 and Bcl-x_{L}) inhibit release, whereas those that promote death (Bax and Bak) induce this release (reviewed in Desagher and Martinou, 2000).

Two competing models have been proposed to explain how proapoptotic activities are released from mitochondria: The nonspecific rupture of the outer mitochondrial membrane, and the formation of conductance channels, which fits better with the absence of mitochondrial morphological changes in many apoptosis models. The ability of some Bcl-2 family members to form ion channels in artificial lipid membranes, together with the fact that Bax and Bak trigger release of cytochrome c when added directly to mitochondria, has led to the hypothesis that Bax and Bak could be involved in formation of conductance channels. Furthermore, Bcl-x_{L}, Bak and Bax can interact with the outer mitochondrial, integral membrane channel protein VDAC. Bcl-x_{L} stimulates closure of the VDAC channel, whereas Bax and Bak facilitate its opening. Moreover Bax and Bak allow cyctochrome c to pass through the VDAC channel (Shimizu et al., 1999).

It was an object of the invention to identify novel genes involved in programmed cell death.

Previous studies have suggested that novel protein synthesis is required for efficient induction of apoptosis in IL-3 dependent myeloid cells (Williams et al., 1990). Therefore, to solve the problem underlying the present invention, in a first step, polysome-bound and polysome free mRNA fractions of FDCP-1 cells, either maintained in IL-3 or withdrawn from IL-3 for 8 hours, were prepared. The polysome-bound fractions were then subjected to suppression subtractive hybridization, SSH, in order to isolate differentially expressed genes.

In the experiments of the present invention, a novel proapoptotic inducer (herein termed "p12") was identified in murine myeloid/erythroid cytokine dependent cells.

It could be shown that the p12 protein is indeed differentially expressed in a cytokine-dependent manner. IL-3 removal induces increased translation of the p12 mRNA. The data obtained in the experiments of the present invention further show that p12, which is localized in the cytosol in the presence of cytokine, translocates into the mitochondrial fraction in the absence of cytokine and binds to a central component (VDAC) of conductance channels in the outer mitochondrial membrane. These results suggest that p12 induces a release of proapoptotic activities from mitochondria in a cytokine dependent manner. From these results it may also be concluded that these activities are most likely involved in the activation of cell type specific caspases in this cell system.

In a first aspect, the present invention relates to the murine p12 polypeptide with the amino acid sequence as set forth in SEQ ID NO:2 or to a polypeptide encoded by a polynucleotide which hybridises under stringent conditions to a polynucleotide having a nucleotide sequence as set forth in SEQ ID NO:1.

In a further aspect, the present invention relates to an isolated DNA molecule comprising a polynucleotide with the nucleotide sequence as set forth in SEQ ID NO:1 encoding murine p12 polypeptide or an isolated DNA molecule encoding murine p12, comprising a polynucleotide which hybridises under stringent conditions to a polynucleotide having a nucleotide sequence as set forth in SEQ ID NO:1.

Based on the information on the murine p12 and the sequence information of EST BF110215, the sequence of the human p12 cDNA and the protein encoded thereby has been obtained.

In a preferred embodiment, the present invention relates to the human p12 polypeptide with the amino acid sequence as set forth in SEQ ID NO:4 or to a polypeptide encoded by a polynucleotide which hybridises under stringent conditions to a polynucleotide having a nucleotide sequence as set forth in SEQ ID NO:3.

In a preferred aspect, the present invention relates to an isolated DNA molecule comprising a polynucleotide with the nucleotide sequence as set forth in SEQ ID NO:3 encoding human p12 polypeptide or an isolated DNA molecule comprising a polynucleotide which hybridises under stringent conditions to a polynucleotide having a nucleotide sequence as set forth in SEQ ID NO:3.

By "stringent hybridisation conditions" as used herein is meant overnight incubation at 42°C in a solution comprising: 50% formamide, 5x SSC (1X SSC = 150 mM NaCl, 15mM trisodium citrate), 50 mM sodium phosphate (pH 7.6), 5x Denhardt's solution, 10% dextran sulfate, and 20 µg/ml denatured, sheared salmon sperm DNA, followed by washing the filters in 0.1x SSC at about 65°C, or equivalent conditions.

Based on the sequence information of EST BF110215, the human homologue of murine p12 is cloned according to standard procedures, e.g. by RT PCR amplification from human RNA extracted from certain tissues or cells (e.g. myeloid cells), using standard PCR protocols. Amplified products are then cloned into suitable vectors for propagation in bacteria, and the DNA sequence of individual amplification products determined and used to identify human p12 homologues according to the criteria defined above.

Functional studies confirming a proapoptotic activity of human p12, corresponding or similar to that of the murine p12, can be done as described in the Examples for the murine p12 in murine myeloid progenitors. Expression, subcellular localization and interaction studies can be done using primary human erythroid progenitors isolated from umbilical cord blood or established human cell lines using antisera directed against the murine p12.

In the following, if not otherwise stated, the term "p12" refers to both the murine and the human p12.

Beside p12 DNA molecules, the present invention relates to isolated p12 nucleic acid molecules in the form of RNA, such as mRNA. The p12 DNA molecules include cDNA and genomic DNA obtained by cloning or produced synthetically. The DNA may be double-stranded or single-stranded. Single-stranded DNA or RNA may be the coding strand, also known as the sense (or plus) strand, or it may be the non-coding strand, also referred to as the antisense (or minus) strand.

By "isolated" nucleic acid molecule(s) is intended a nucleic acid molecule, DNA or RNA, which has been removed from its native environment.

The present invention also relates to p12 variants, precursors, fragments or derivatives, which may include one or more polynucleotides having a nucleic acid sequence that is complementary to substantially all or a portion of a nucleic acid molecule having a nucleic acid sequence as shown in SEQ ID NO:1 or SEQ ID NO:3. As used herein, the terms "a portion" or "a fragment" of a nucleic acid molecule or a polypeptide means a segment of a polynucleotide comprising at least 15 nucleotides (nt), usually at least 18 nt or 25 nt, and may be at least about 50 nt contiguous nucleotides or amino acids of a reference polynucleotide or polypeptide (for example, the polypeptide and polynucleotide shown in SEQ ID NO: 1 or SEQ ID NO:3, or SEQ ID NO:2 or SEQ ID NO:4, respectively).

Double or single stranded p12 DNA fragments may be obtained by chemically synthesizing oligonucleotides in accordance with conventional methods, by restriction enzyme digestion, by PCR amplification, external deletions, etc. Such small DNA fragments are useful as primers for PCR, hybridization screening probes, etc. Larger DNA fragments, greater than 100 nt are useful for production of the encoded polypeptide. For use in amplification reactions, such as PCR, a pair of primers will be used. The exact composition of the primer sequences is not critical to the invention, but for most applications the primers will hybridize to the subject sequence under stringent conditions, as known in the art. It is preferable to choose a pair of primers that will generate an amplification product of at least about 50 nt, preferably at least about 100 nt. Algorithms for the selection of primer sequences are generally known, and are available in commercial software packages. Amplification primers hybridize to complementary strands of DNA, and will prime towards each other.

Preferably, the invention relates to p12 DNA fragments that can be used for producing functionally active p12 fragments, which are useful in therapy. To design such fragments, the following experimental approach may be taken:

In a first step, p12 cDNA fragments of varying length are obtained by external deletion mutagenesis according to standard protocols, e.g. by commercially available tools (Erase-a-Base, Promega). The fragments are then, as described below for the p12 versions with modified proapoptotic activity, cloned into suitable expression vectors, expressed in test cells and assessed for the desired proapoptotic activity, e.g. in the TUNEL assay.

Alternatively, p12 fragments may be designed based on secondary structure predictions.

Secondary structure predictions using standard software (PredictProtein) suggest that the murine and human p12 proteins are mostly alpha-helical in the N-terminus from amino acids 1-84. In detail, predicted alpha helical domains extend from amino acids (aa) 1-4, aa 8-19, aa 34-44, aa 52-84. The C-terminus from amino acids 85-113 is predicted to contain mainly coiled structure.

Based on the secondary structure, two primers representing the borders of the domain of interest, e.g. one of the α-helical domains, are used for PCR amplification. The thus obtained fragment is subcloned and further tested for proapoptotic activity as described above.

Besides the DNA molecules having a nucleotide sequence corresponding to that depicted SEQ ID NO:1 or SEQ ID NO:3; the invention also relates to DNA molecules which comprise a sequence substantially different from those described above but which, due to the degeneracy of the genetic code, still encode the p12 mouse or human polypeptides. Since the genetic code is well known in the art, it is routine for one of ordinary skill in the art to produce the degenerate variants described above without undue experimentation.

In addition, the invention relates to p12 polypeptides which have deviations from the sequence shown in SEQ ID NO:2 or SEQ ID NO:4, caused by the conservative exchange of amino acids, if they are p12 derivatives or fragments or peptides with the properties which are desirable for their use in therapy or in screening assays described below. The invention also relates to isolated DNA molecules encoding such derivatives with a polynucleotide sequence varying in their sequence from SEQ ID NO:1 or SEQ ID NO:3.

Further examples of isolated DNA molecules include recombinant DNA molecules maintained in heterologous host cells, and those DNA molecules purified (partially or substantially) from a solution whether produced by recombinant DNA or synthetic chemistry techniques. Isolated RNA molecules include *in vivo* or *in vitro* RNA transcripts of the DNA molecules of the present invention. However, it is intended that "isolated" as used herein does not include the p12 cDNA present in a cDNA library or in a preparation of purified or isolated genomic DNA containing the p12 gene or a portion thereof in admixture with one or more other cDNA molecules or DNA fragments.

In a further aspect, the present invention relates to isolated genomic p12 DNA molecules containing, besides the coding sequence, the regulatory elements including the promotor sequence.

The genomic p12 DNA can be obtained as follows: genomic clones, e.g. cosmid clones, can be isolated according to standard procedures using the p12 cDNA, or a fragment thereof, as a hybridization probe. Individual subfragments of the obtained clones are tested in transcriptional reporter assays by using expression constructs containing a reporter gene, e.g. lacZ or luciferase, under the control of a minimal promotor sequence, e.g. from the CMV minimal promotor. The subfragments of interest are inserted 5' of the minimal promotor and their effect on reporter gene expression is tested in hematopoietic cells. In a step by step process, further deletions of the subfragments are used to determine the minimal functional promotor sequence.

In a further aspect, the invention relates p12 DNA or polypeptide homologues, which have been obtained by mutagenesis.

The p12 DNA, including flanking promoter regions and coding regions, may be mutated in various ways known in the art to generate targeted changes in promoter strength, sequence of the encoded protein, etc. The DNA sequence or protein product of such a mutation will usually be substantially similar to the sequences as shown in SEQ ID NO:1 or SEQ ID NO:3, or SEQ ID NO:2 or SEQ ID NO:4, i.e. it will differ by at least one nucleotide or amino acid, respectively, and may differ by at least two but not more than about ten nucleotides or amino acids. The sequence changes may be substitutions, insertions or deletions. Deletions may further include major changes, such as deletions of a domain or exon.

Homologues of the p12 proteins also include versions of the polypeptide containing single or multiple point mutations and/or insertions and/or deletions which are resistant to or mimic post-translational modification. Such mutations may effect the activity or subcellular localisation of the protein. The mutations may be carried out according to standard protocols, e.g. by means of commercially available kits (e.g. QuickChange, Stratagene), the obtained mutated p12 cDNA is then inserted into vectors suitable for transfection of test cells, e.g. hematopoietic or other cells in which wildtype p12 plays an important physiological role. Examples for suitable vectors are retroviral vectors. Upon expression of p12, the proapoptotic activity is assessed in the test cells by standard viability assays, e.g. the TUNEL assay.

In addition, the subcellular localization of the mutated p12 may be assessed by immunofluorescence procedures, i.e. using anti-p12 antibodies and secondary fluorescence-labeled antibodies according to standard protocols. A mutated protein which has a reduced proapoptotic activity and a subcellular localization different from that of the wildtype p12 may significantly inhibit the proapoptotic activity of the endogenous p12 by its sequestration or competition with binding partners. Such p12 mutants are useful for therapy of conditions in which increased cell death is involved, e.g. myelodysplastic syndromes.

Alternatively, mutants with enhanced proapoptotic activity may be obtained.

Such mutants may exert their effect by being constitutively localized to mitochondria and can thus be used for the treatment of conditions in which reduced cell death is involved, e.g. cancerous conditions and leukemias.

Nucleic acid molecules of the present invention which encode a p12 polypeptide or a variant, derivative or fragment thereof ("p12 nucleic acid molecules") further include those encoding the amino acid sequence of the polypeptide by itself, together with additional, non-coding sequences, including for example introns and non-coding 5' and 3' sequences, such as the transcribed, untranslated regions (UTRs) or other 5' flanking sequences that may play a role in transcription (e.g. via providing ribosome- or transcription factor-binding sites), mRNA processing (e.g. splicing and polyadenylation signals) and stability of mRNA; the coding sequence for the p12 polypeptide operably linked to a regulatory DNA sequence, particularly a heterologous regulatory DNA sequence such as a promoter or enhancer; and the coding sequence for the p12 polypeptide linked to one or more sequences which code for amino acids that provide additional functionalities. By way of example, the DNA encoding the p12 polypeptide may be fused to a marker sequence, such as a sequence encoding a peptide which facilitates purification of the fused polypeptide; e.g. modifications in the form of epitope tagging. In certain embodiments of this aspect of the invention, the marker amino acid sequence may be a hexa-histidine peptide, such as the tag provided in a pQE vector (Qiagen, Inc.), among others, many of which are commercially available. The "HA" tag is another peptide useful for purification which corresponds to an epitope derived from the influenza hemagglutinin protein, which has been described by Wilson et al., 1984. Yet another useful marker peptide for facilitation of purification of p12 is glutathione S-transferase (GST) encoded by the pGEX fusion vector (see, e.g., Winnacker, 1987). Other useful tag system is the FLAG system (Jarvik and Telmer, 1998). For studies of subcellular localization, fusion proteins with green fluorescent proteins (GFPs) may be used.

The above-described p12 polypeptides, variants, derivatives or fragments thereof may be expressed in prokaryotic or eukaryotic expression systems according to well-established methods.

The p12 nucleic acids can also be used for identifying homologous or related genes; in identifying compounds that modulate the expression or function of the p12 protein; mapping functional regions of the protein; and in studying of apoptosis pathways and associated physiological pathways (as described below).

The p12 promotor sequence is useful for determining whether, in a patient, reduced or absent p12 expression correlates with a mutation in the promotor sequence. To this end, genomic DNA is isolated from patient material, the p12 promotor region is amplified by PCR and the sequence compared with the authentic promotor sequence from healthy individuals.

The p12 promotor is also useful in screening assays for identifying drugs that modulate, e.g. upregulate, p12 expression at a transcriptional level. Typically, such an transcriptional assay uses hematopoietic cells, which have been transfected with a reporter construct in which the reporter gene is under the control of the p12 promotors. The cells are incubated, in the absence or presence of a test substance, and the effect of the substance on reporter gene expression is measured. A substance upregulating reporter gene expression is a candidate for upregulating p12 transcription.

Transcriptional assays of this type are well-known in the art, they have been described, inter alia, in WO 92/13063.

Alternatively, cell-based screening assays in microtiter format can be used to identify drugs that inhibit interaction of p12 with critical interaction partners required for apoptosis induction (e.g.: VDAC, see Example 3). Fusion proteins of the interaction partners of interest, e.g p12 and VDAC, are expressed in yeast or mammalian cells; interaction of the binding partners leads to expression of an easily detectable marker (e.g.: GFP) or a resistance gene product (e.g.: Ura3, neomycinR) that allows cell proliferation under selective conditions, depending on the particular system used. Drugs interfering with the interaction will lead to loss of marker expression or loss of cellular proliferation. Standard two hybrid interaction systems using yeast or mammalian cells are of particular use (Fotin-Mleczek et al., 2000).

In another approach the screening method may be based on a cell-free assay.

The principle of such an assay is to inhibit the physical interaction of a protein of interest with its binding partner. The assay may be conducted by incubating the binding partners in solution, one of them being fused to a reporter gene product (e.g. alkaline phosphatase or luciferase, which allows for monitoring the interaction by determining the enzymatic activity) and the other one being immobilized on a suitable carrier, e.g. micro titer plates, with the test substance and by determining the effect of a drug on the interaction of the two proteins by measuring the reporter activity. One binding partner may carry a tag resulting from the antecedent purification step, e.g. a GST, His or Myc tag, provided binding of the partners is not impaired by the tag. In this case, one partner may be specifically immobilized on the carrier via that tag. If a substance is an inhibitor of the interaction, the reporter signal is decreased or abolished after washing off the carrier. Alternatively, larger proteins containing the binding domains may be used, e.g. the full length proteins, or one of the interaction partners is used as a full-length protein and the other one in the form of the binding domain only.

Alternatively, one of the interaction partners may be labeled with any other detectable marker, preferably a marker producing a signal that can be easily measured in a high throughput screen, in which the unlabeled interaction molecule is immobilized, e.g. on a microtiter plate surface or on beads. Examples for suitable labels are radioactive labels or commercially available fluorescent markers suitable for labeling proteins or peptides.

For a cellular screening assay method, cytokine dependent hematopoietic cells in combination with viability assays (MTT assays, luciferase assay; Promega) that allow high throughput screening may be employed.

The therapeutic utility of a compound that mimics the action of p12 in apoptosis induction, e.g. by binding to VDAC, or that increases p12 expression, is confirmed by measuring its effect on endogenous p12 expression by means of Northern Blotting and/or conventional nuclear run-on assays.

Modulation of the p12 gene activity *in vivo,* e.g. upregulation of p12 activity by applying a drug which has been identified in the above-described screening assay, is useful for prophylactic and therapeutic purposes, such as treatment of cancer and other hyperproliferative disorders (like myeloid and erythroid leukemias, where p12 has been shown to exert a proapoptotic activity). The murine p12 mRNA demonstrates a tissue specific expression pattern with highest levels observed in heart, liver, kidney, brain and testes and may play a similar proapoptotic role in these tissues as it plays in the hematopoetic system. Due to the high conservation between the murine and the human p12 protein, the human protein is expected to have a corresponding function, which may be confirmed as described above.

The genomic sequences surrounding the p12 locus may be used to identify *cis* acting elements required for transcriptional or translational regulation of p12 expression, especially in different tissues or stages of development, and to identify *cis* acting sequences and *trans* acting factors that regulate or mediate p12 expression. Such transcription or translational control regions may be operably linked to a p12 gene in order to promote expression of wild type or altered p12 or other proteins of interest in cultured cells, or in embryonic, fetal or adult tissues, and for gene therapy. Naturally occurring polymorphisms in the promoter region are useful for determining natural variations in expression, particularly those that may be associated with disease.

The p12 DNA may also be used to identify expression of the gene in a biological specimen. The manner in which cells can be probed for the presence of particular nucleotide sequences, as genomic DNA or RNA, is well established in the literature; briefly, DNA or mRNA is isolated from a cell sample. Next, the mRNA is amplified, e.g. by RT-PCR, using reverse transcriptase to form a complementary DNA strand, followed by polymerase chain reaction amplification using primers specific for the subject DNA sequences. Alternatively, the mRNA sample is separated by gel electrophoresis, transferred to a suitable support, e.g. nitrocellulose, nylon, etc., and then probed with a fragment of the subject DNA as a probe. Other techniques, such as oligonucleotide ligation assays, *in situ* hybridizations, and hybridization to DNA probes arrayed on a solid chip may also find use. Detection of mRNA hybridizing to the subject sequence is indicative of p12 gene expression in the sample.

The p12 DNA molecules are useful in gene therapy to treat disorders associated with a deficit in pro-apoptosis proteins, including different states of tumorgenesis. The p12 DNA molecules of the present invention may be administered, preferably in recombinant form as plasmids, directly or as part of a recombinant virus.

Expression vectors generally have convenient restriction sites located near the promoter sequence to provide for the insertion of nucleic acid sequences. Transcription cassettes may be prepared comprising a transcription initiation region, the p12 DNA or fragment thereof, and a transcriptional termination region. The transcription cassettes may be introduced into a variety of vectors, e.g. a plasmid; retrovirus, vaccinia virus, adenovirus, listeria monocytogenes; and the like (a summary was provided by Coulie, 1997).

Moreover, synthetic carriers for nucleic acids such as cationic lipids, microspheres, micropellets or liposomes may be used for *in vivo* administration of a p12 nucleic acid. The application may optionally be combined with physical methods, e.g. electroporation. p12 antisense molecules can be used to down-regulate expression of p12 in cells or tissues with excessive cell death (as is observed in e.g.: myelodysplastic syndrome). The antisense reagent may be antisense oligonucleotides (ODN), particularly synthetic ODN having chemical modifications from native nucleic acids, or nucleic acid constructs that express such anti-sense molecules as RNA. The antisense sequence is complementary to the mRNA of the targeted gene, and inhibits expression of the targeted gene products. Antisense molecules inhibit gene expression through various mechanisms, *e.g.* by reducing the amount of mRNA available for translation, through activation of RNAse H, or steric hindrance. One or a combination of antisense molecules may be administered, where a combination may comprise multiple different sequences.

p12 antisense oligonucleotides may be chemically synthesized by methods known in the art (see Wagner et al., 1996). Preferred oligonucleotides are chemically modified from the native phosphodiester structure, in order to increase their intracellular stability and binding affinity. A number of such modifications have been described in the literature, which alter the chemistry of the backbone, sugars or heterocyclic bases.

Among useful changes in the backbone chemistry are phosphorothioates; phosphorodithioates, where both of the non-bridging oxygens are substituted with sulfur; phosphoroamidites; alkyl phosphotriesters and boranophosphates. Achiral phosphate derivatives include 3'-O'-5'-S-phosphorothioate, 3'-S-5'-O-phosphorothioate, 3'-GH2-5'-O-phosphonate and 3'-NH-5'-O-phosphoroamidate.

Peptide nucleic acids replace the entire ribose phosphodiester backbone with a peptide linkage. Sugar modifications are also used to enhance stability and affinity. The α-anomer of deoxyribose may be used, where the base is inverted with respect to the natural 3-anomer. The 2'-OH of the ribose sugar may be altered to form 2'-O-methyl or 2'-O-allyl sugars, which provides resistance to degradation without comprising affinity. Modification of the heterocyclic bases must maintain proper base pairing. Some useful substitutions include deoxyuridine for deoxythymidine; 5-methyl-2'-deoxycytidine and 5-bromo-2'-deoxycytidine for deoxycytidine. 5-propynyl-2'-deoxyuridine and 5-propynyl-2'-deoxycytidine have been shown to increase affinity and biological activity when substituted for deoxythymidine and deoxycytidine, respectively.

The nucleic acids of the invention, e.g. modified murine or human p12 cDNAs or p12 cDNA fragments, can also be used to generate transgenic animals or site specific gene modifications in animals or cell lines derived from the transgenic animals. Transgenic animals, which are useful for *in vivo* studies of p12 derivatives or fragments, may be obtained through homologous recombination, where the normal p12 locus is altered. Alternatively, a nucleic acid construct is randomly integrated into the genome. Vectors for stable integration include plasmids, retroviruses and other animal viruses, YACs, and the like. Transgenic animals are also useful for verifying that a sequence identified as a minimal promotor sequence also functions in vivo and confers a similar or identical expression pattern as the genomic p12 locus.

The modified cells or animals are useful in the study of pro-apoptotic gene function and regulation. For example, a series of small deletions and/or substitutions may be made in the p12 gene to determine the role of different epitopes in oncogenesis, signal transduction, etc. p12 is also useful to construct transgenic animal models for proliferative disorders, e.g. leukemia, where expression of p12 is specifically reduced or absent. Specific constructs of interest include anti-sense p12, which will block p12 expression, and expression of dominant negative p12 mutations. A detectable marker, such as *lac Z* may be introduced into the p12 locus, where upregulation of p12 expression will result in an easily detected change in phenotype.

One may also provide for expression of the p12 DNA or variants thereof in cells or tissues where it is not normally expressed or at abnormal times of development. By providing expression of p12 protein or variants thereof in cells in which it is not normally produced, one can induce changes in cell behavior, e.g. increased or decreased cell death.

DNA constructs for homologous recombination will comprise at least a portion of the p12 gene with the desired genetic modification, and will include regions of homology to the target locus. Conveniently, markers for positive and negative selection are included. Methods for generating cells having targeted gene modifications through homologous recombination are known in the art. For various techniques for transfecting mammalian cells, see Keown et al., 1990.

For embryonic stem (ES) cells, an ES cell line may be employed, or embryonic cells may be obtained freshly from a host, e.g. mouse, rat, guinea pig, etc. Such cells are grown on an appropriate fibroblast-feeder layer or grown in the presence of leukemia inhibiting factor (LIF). When ES or embryonic cells have been transformed, they may be used to produce transgenic animals. After transformation, the cells are plated onto a feeder layer in an appropriate medium. Cells containing the construct may be detected by employing a selective medium. After sufficient time for colonies to grow, they are picked and analyzed for the occurrence of homologous recombination or integration of the construct. Those colonies that are positive may then be used for embryo manipulation and blastocyst injection. Blastocysts are obtained from 4 to 6 week old superovulated females. The ES cells are trypsinized, and the modified cells are injected into the blastocoel of the blastocyst. After injection, the blastocysts are returned to each uterine horn of pseudopregnant females. Females are then allowed to go to term and the resulting offspring screened for the construct. By providing for a different phenotype of the blastocyst and the genetically modified cells, chimeric progeny can be readily detected.

The chimeric animals are screened for the presence of the modified gene and males and females having the modification are mated to produce homozygous progeny. Homozygous null mutants or other mutants may be crossed to mice carrying deletions or mutations in other apoptotic regulators to study genetic interactions. If the gene alterations cause lethality at some point in development, tissues or organs can be maintained as allogeneic or congenic grafts or transplants, or in *in vitro* culture. The genetically modified animals may be any non-human mammal, such as laboratory animals, domestic animals, etc. The genetically modified animals may be used in functional studies, drug screening, phenotypic analyses, etc.

The availability of a number of proteins that are players in the apoptotic apparatus allows *in vitro* reconstruction of the apoptosis pathway. Two or more of the components, including p12, may be combined *in vitro,* and the behavior assessed in terms of activation of cellular organelles that play a central role in apoptosis (like mitochondria); modification of protein components, e.g. proteolytic processing, phosphorylation, methylation, etc.; ability of different protein components to bind to each other, etc. The components may be modified by sequence deletion, substitution, etc. to determine the functional role of specific domains.

The p12 nucleic acid sequences may be used to analyze a patient sample for the presence of polymorphisms associated with a disease state or genetic predisposition to a disease state. Biochemical studies may be performed to determine whether a sequence polymorphism in a p12 coding region, e.g. an SNP (Single Nucleotide Polymorphism) or control regions is associated with the disease. Disease-associated polymorphisms may include deletions, insertions or truncation in the gene, mutations that alter expression level, etc.

Changes in the promoter or enhancer sequence that may affect expression levels of p12 can be compared to expression levels of the normal allele by various methods known in the art. Methods for determining promoter or enhancer strength include quantitation of the expressed natural protein; insertion of the variant control element into a vector with a reporter gene such as β-galactosidase, luciferase chloramphenicol acetyltransferase, etc. that provides for convenient quantitation; and the like.

A number of methods are available for analyzing nucleic acids for the presence of a specific sequence, e.g. a disease associated polymorphism. Where large amounts of DNA are available, genomic DNA is used directly. Alternatively, the region of interest is cloned into a suitable vector and grown in sufficient quantity for analysis. Cells that express p12 may be used as a source of mRNA, which may be assayed directly or reverse transcribed into cDNA for analysis. The nucleic acid may be amplified by conventional techniques, such as the polymerase chain reaction (PCR), to provide sufficient amounts for analysis. The use of the polymerase chain reaction is described in Saiki, et al., 1985, and a review of techniques may be found in Sambrook, et al., 1989. Alternatively, various methods are known in the art that utilize oligonucleotide ligation as a means of detecting polymorphisms, for examples see Riley et al., 1990, and Delahunty et al., 1996.

The p12 protein is also useful as an immunogen for producing specific antibodies.

Thus, a still further aspect of the present invention relates to antibodies and antibody preparations specifically reactive with an epitope of the p12 polypeptide.

Polyclonal anti-p12 antibodies are conventionally obtained by immunising animals, particularly rabbits and goats, by injecting the p12 antigen or fragments thereof and subsequently purifying the immunoglobulin.

Monoclonal anti-p12 antibodies may be obtained by standard procedures following the principle described by Köhler and Milstein, 1975, by immunising animals, particularly mice, then immortalising antibody-producing cells from the immunised animals, e.g. by fusion with myeloma cells, and screening the supernatant of the hybridomas obtained by immunological standard assays for monoclonal anti-p12 antibodies. For therapeutic or diagnostic use in humans, these animal antibodies may optionally be chimerised in the conventional way (Neuberger et al., 1984; Boulianne et al., 1984) or humanised (Riechmann et al., 1988; Graziano et al., 1995).

Antibodies specific for a p12 may be used in staining or in immunoassays of samples from an individual to be analysed for p12 and for screening human conditions for P12 mediated pathologies. Samples used in these assays include biological fluids such as semen, blood, cerebrospinal fluid, tears, saliva, lymph, dialysis fluid and the like; organ or tissue culture derived fluids; and fluids extracted from physiological tissues. Also included are derivatives and fractions of such fluids. The cells may be dissociated, in the case of solid tissues, or tissue sections may be analyzed. Alternatively a lysate of the cells may be prepared.

In the following Examples, the identification and characterization of the novel proapoptotic p12 activity in cytokine dependent myeloid/erythroid progenitors is described.

### Example 1

### Screen for novel genes involved in induction of apoptosis in hematopoietic cells: combining polysome gradients and suppression subtractive hybridization (SSH)

A screening procedure was developed to identify potential proapoptotic activities in myeloid IL-3 dependent progenitors. The screen is based on previous data suggesting that novel protein synthesis is required for proper induction of apoptosis in this cell system (Williams et al., 1990). Goal of the screen is to identify mRNAs that are translated preferentially in cytokine depleted cells as compared to cells grown in the presence of cytokine. It is pointed out that under these experimental conditions the bulk of cellular transcription and translation drops.

Identification of the genes of the present invention was done by using polysome-bound mRNA preparations for differential screening, allowing to simultaneously assess differential expression of genes at multiple, important levels, i.e. transcriptional control, mRNA processing, nuclear export, mRNA stability and translational control.

This method for identifying and isolating genes differentially expressed with respect to protein levels produced, comprises the following steps:
a) preparing cytosolic extracts from two (or more) different cell populations to be compared
b) separating the cytosol (e.g. on sucrose gradients) to obtain fractions containing either the ribosome-free mRNA compartment or mRNA bound to polyribosomes of increasing size.
c) isolating free and polysome bound mRNA from adequately pooled sucrose gradient fractions
d) preparing DNAs complementary to the mRNAs defined in c)
e) subjecting the c-DNAs to the different procedures available for differential screening, i.e. by PCR-based subtractive hybridization methods such as suppression subtration hybridization (SSH) or hybridizing the cDNAs with arrays or chips of multiple known or unknown c-DNAs
f) identifying differentially expressed genes (c-DNAs) by hybridizing them to control preparations of polysome-bound versus free mRNAs from the cell types to be compared and determining the differences in the abundance of mRNA in the compartments.
g) selectively amplifying and cloning the cDNAs identified in step f).

IL-3 dependent murine myeloid progenitor FDCP-1 cells were used in this screen. Cells were grown in RPMI medium (Beug et al., 1995) or serum-free StemPro34™ (Life Technologies) according to the instructions of the supplier. IL-3 was added as recombinant protein (murine recombinant IL-3, Pepro Tech, 1 ng/ml). Cytokine depletion was done as follows: after 200x10⁶ cells had been obtained, one aliquot of 100x10⁶ cells was washed twice with PBS and reseeded in medium without IL-3 for 8 hours. The other aliquot was maintained in IL-3 for the same time.

Lysis of 100 x 10⁶ cells from both aliquots for sucrose gradient centrifugation and analysis of the gradients was done as follows:

Cells were lysed at 4°C in extraction buffer containing 10 mM Tris-HCl (pH 8.0), 140 mM NaCl, 1.5 mM MgCl₂, 0.5% Nonidet-P-40, 500 U/ml RNasin, 20 mM dithiothreitol, 150 µg/ml cycloheximide and 1 mM phenylmethylsulfonyl fluoride. Nuclei were removed by centrifugation at 3000 g for 2 min at 4°C. The supernatant was further supplemented with 665 µg/ml heparin, centrifuged at 12000 g for 10 min at 4°C to eliminate mitochondria and layered onto a 10 ml linear sucrose gradient (15-40% sucrose [w/v] supplemented with 10 mM dithiothreitol, 100 µg/ml cycloheximide and 0.5 mg/ml heparin). Centrifugation was performed in a SW41Ti rotor at 38000 rpm for 120 min at 4°C. Fractions of 550 µl were harvested and subjected to proteinase K digestion by adding 1% sodiumdodecylsulfate (SDS), 10 mM EDTA and 200 µg/ml proteinase K for 30 min at 37°C. After phenol extraction and ethanol precipitation, the RNA was analyzed by electrophoresis on denaturing 1% formaldehyde agarose gels and subsequent Northern blotting. After UV-crosslinking of RNA to the nylone membranes (GeneScreen, DuPont), the distribution of 18S and 28S rRNA was visualized by staining of filters with methylene blue. The emergence of a constant molar ratio between 28S and 18S ribosomal RNA within one fraction indicates the joining of the 80S initiation complex to the mRNAs and marks the border between the ribosome-free and ribosome-bound compartment (Peng et al., 1996). Alternatively, this boundary may be visualized by hybridization with a [³²P]-labeled probe specific for the 28S ribosomal RNA. In most cases, RNA fractions 1-8 or 1-9 (depending on the cell type, see examples) of each sucrose gradient, representing the ribosome-free RNA population and RNA fractions 11-20 or 12-20, corresponding to the ribosome-bound RNA compartment, were pooled and subjected to poly(A)⁺ mRNA selection. Only polysome-bound mRNA fractions were used for further steps of the differential screening. Extracts from cells maintained in IL-3 or withdrawn from IL-3 for 4 and 8 hours were also fractionated on sucrose gradients, for these, however, mRNA was separately isolated from each fraction to verify translational control of candidate genes isolated in the SSH screen.

Poly(A)⁺ mRNA of unbound and polysome-associated RNA species was selected using the Oligotex-dT purification system (Quiagen), however, poly(A)⁺ mRNA selection can be performed with various poly(A)⁺ mRNA isolation procedures within kits provided by different manufacturers. Poly(A)⁺ mRNA of highest quality must be sufficiently enriched in terms of decreasing the portion of ribosomal RNA in the poly(A)⁺ mRNA preparation to <20% when compared to the portion of ribosomal RNA present in the cytoplasmic RNA preparation. poly(A)⁺ mRNA is quantified by standard OD₂₆₀-reading. Limitations: at least 0.3-0.4 µg of poly(A)⁺ mRNA is required for cDNA synthesis described in this protocol.

Subtractive hybridization using polysome bound mRNA as a tester, and amplification of targets was performed as described in the Clontech PCR-Select cDNA Subtraction Kit. PCR fragments obtained from the screen were isolated from 1% agarosegel using Quiagen QIAquick Gel Extraction Kit and subsequently cloned into the lacZ sequence of Promega pGEM-T Vector following protocol of the manufacturer. This allowed blue/white screening for recombinants. Plasmid DNA of the white colonies was isolated and digested with NcoI / SalI endonucleases to check for inserts and sequenced by standard methods.

BLAST searches were performed on the following databases: Genebank non-redundant cds, EST databases, Drosophila genome database, C.elegans genome database.

The initial SSH screen of polysome-bound mRNA from FDCP-1 cells maintained in IL-3 or withdrawn from IL-3 for 8 hours (using the RNA from the IL-3 depleted cells as tester) yielded 6 known genes and 11 EST sequences with no homology in the database. Out of these, two known genes and one EST showed clear differential expression after rescreening by Northern blots. One of these genes was previously known to be involved in apoptosis ("requiem", Gabig et al., 1994) therefore it was not further characterized. The second gene, cyclophilin, is a potential member of the permeability transition complex of mitochondria, its role in apoptosis in hematopoietic cells however is not yet clear. The third differentially regulated sequence herein designated p12, had no homology to any known gene or described gene functions. A full length cDNA of p12 was produced by 5' RACE using purified mRNA from FDCP-1 cells and the SMART RACE cDNA Amplification kit. Cloning and sequencing of this gene revealed an open reading frame of 12 kD. A number of identical sequences were found in the mouse EST databases. Significant homology of the mouse p12 gene was found to ESTs from human (e.g.: AAF28946.1) and rat (e.g.: AA892572), representing approximately 60% sequence identity at the protein level. Similar sequences were also present in Drosophila (AE003682) and C. elegans (P34341) genome databases, suggesting the existence of homologs with similar functions in invertebrates.

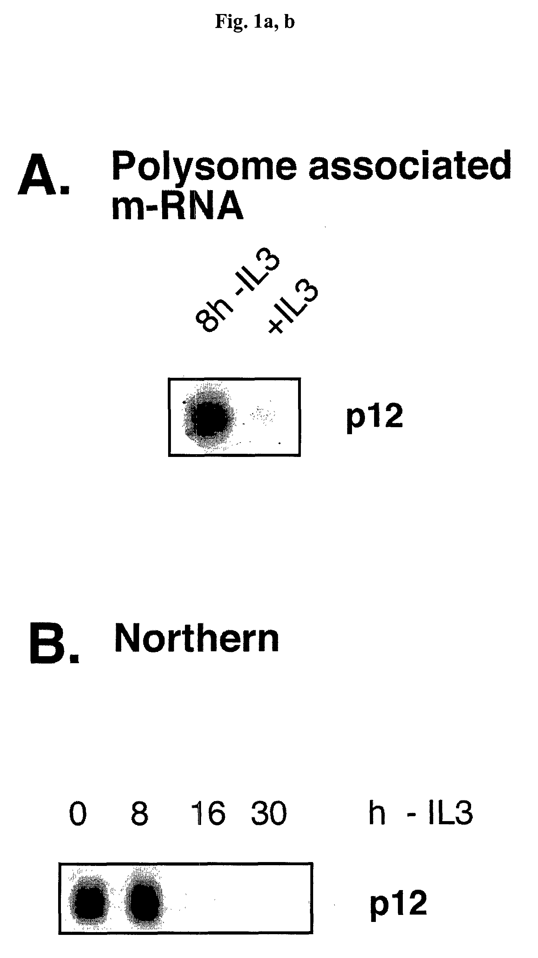

Probes for further northern blot analysis were prepared from gel purified cDNA fragments using Gibco/Life Technologies Random Primers Labeling System according to instructions of the manufacturer. The candidate genes isolated from the SSH screen were first tested by Northern blot, using combined polysome-bound fractions of mRNA from FDCP-1 cells maintained in IL-3 or withdrawn from IL-3 for 8 hours. Out of the 4 mRNAs that were differentially expressed, the p12 mRNA appeared to be significantly regulated at the translational level since it was significantly enriched in the polysome bound fraction of IL-3 deprived cells (Fig. 1A). In contrast, the cDNA hybridized equally well to total mRNA from FDCP-1 cells maintained in IL-3 or withdrawn from IL-3 for 8 hours. Only 16 hours after IL-3 withdrawal, where most cells had already entered apoptosis, synthesis of the total cellular mRNA was downregulated (Fig. 1B).

To verify translational regulation of the p12 mRNA, the labelled cDNA was hybridized to a Northern blot containing the mRNAs from individual fractions of polysome gradients from FDCP-1 cells withdrawn from IL-3 for 4 and 8 hours as well as those maintained in IL-3 (- IL-3, 0h, see Fig. 1C). Already 4 hours after IL-3 withdrawal, a significant fraction of the mRNA had shifted from the free mRNA pool or from monosomes to the polysome pool. This effect was even stronger after 8 hours of IL-3 withdrawal. As expected, a control mRNA from a housekeeping gene (GAPDH) did not undergo any shift from the free to the polysome bound mRNA fractions (Fig. 1C).

### Example 2

### Characterization of p12 protein expression

Antisera directed against p12 were produced by injecting rabbits with KLH coupled to a 12 amino acid long carboxy-terminal peptide whose sequence was derived from the predicted ORF of the murine p12 cDNA. Injection was repeated four times in two weeks intervals.

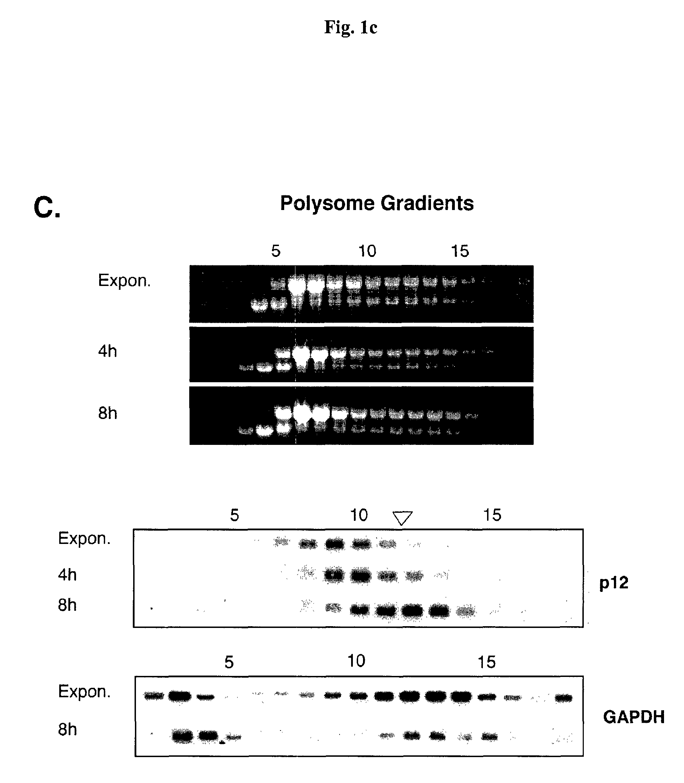

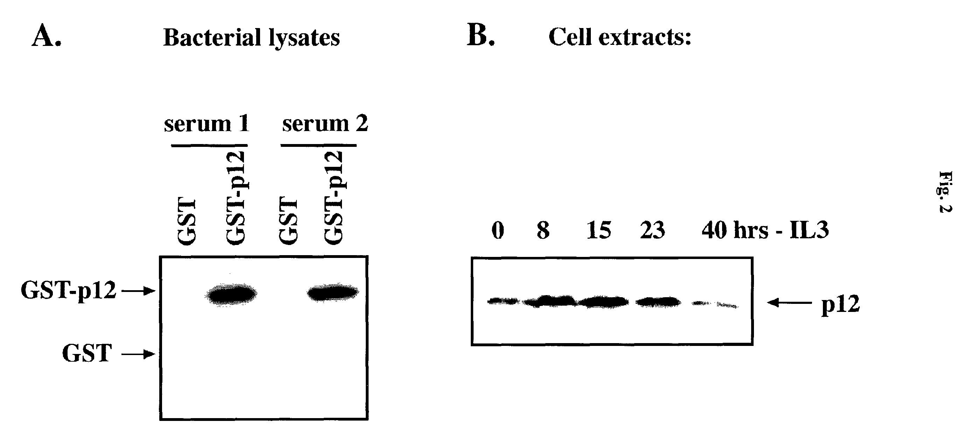

Antisera were first tested on the bacterially produced GST-p12 fusion protein containing the full length p12 fused to the C-terminus of GST. In bacterial cell lysates, the antisera specifically recognize the GST-p12 fusion but not GST alone (Fig. 2A). In order to verify that expression of the p12 protein was indeed induced in FDCP-1 cells by IL-3 withdrawal, western blots from cell lysates of FDCP-1 cells maintained in IL-3 or withdrawn from IL-3 for different time intervals were probed with anti-p12 antisera. Fig. 2B demonstrates that a 12kd protein is weakly expressed in the proliferating cells, but upregulated 8 hours after IL-3 withdrawal.

### Example 3

### Functional characterization of p12

The p12 gene product was further analyzed at the functional level, to determine if it had a role in apoptosis induced by cytokine withdrawal. For this, the cDNA was introduced into a retroviral vector allowing conditional expression via a tetracyclin/doxycylin inducible Tet-repressor-VP16 fusion protein (Paulus et al., 1996; retro-Tet-off vector, Clontech).

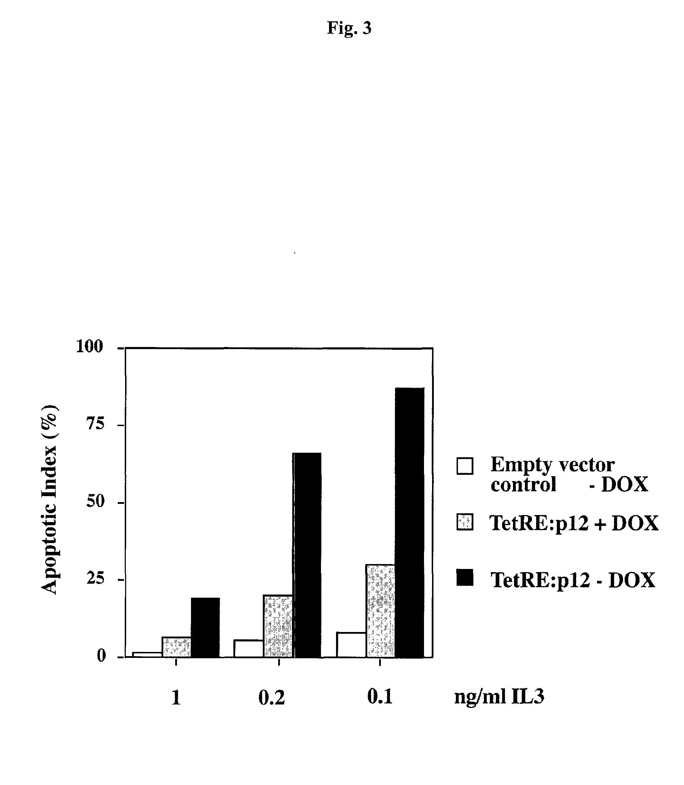

Infected FDCP-1 cell clones were maintained in supersaturating concentrations of IL-3 and in the presence of doxycylin, which represses expression of p12 from the retroviral construct, in order to protect cells from the potential pro-apoptotic activity of p12. To induce expression of p12, doxycylin was removed from the medium and viability in the cultures was measured using the TUNEL assay. As a control clones infected with empty retrovirus were used under identical experimental conditions. p12 expressing clones died after removal of doxycyclin (Fig. 3, black bars). The degree of apoptosis induction was dependent on IL-3 concentration (1 ng/ml to 0.1 ng/ml) and almost complete at the lowest concentration tested (0.1 ng/ml). In the presence of doxycylin, induction of apoptosis was seen at an intermediate level, due to the leakiness of the promoter (Fig. 3, grey bars). Control clone cultures contained very few apoptotic cells even under low IL-3 concentrations (Fig. 3, white bars).

These data demonstrate that p12 expression sensitizes myeloid IL-3 dependent cells to apoptosis under limiting cytokine concentration in a dose dependent manner, suggesting that the gene product is a pro-apoptotic regulator in FDCP cells.

It was also investigated whether p12 induces apoptosis in other cell types. Mouse fibroblasts were transiently transfected using Lipofectamine (Gibco BRL) with an expression construct co-expressing green fluorescent protein (GFP) and the p12 cDNA. 24 and 48 hours after transfection, the cells were analyzed for GFP expression and apoptosis induction using the TUNEL assay by FACS analysis. No induction of apoptosis was detected in the GFP-expressing cells at either time point (data not shown). Together with the tissue specific expression of p12 mRNA (not shown) these results suggest that the p12 protein induces apoptosis in a cell type specific manner.

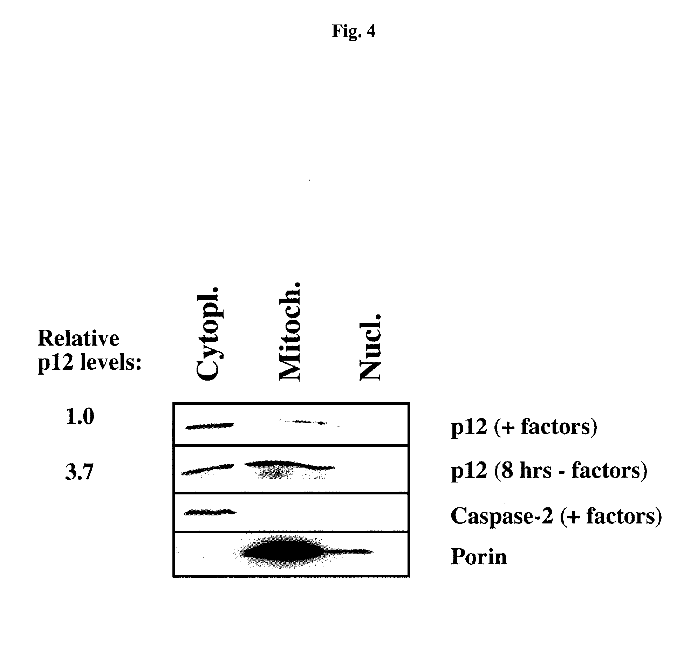

To address the intracellular localization of p12, cell lysates from exponentially growing and cytokine deprived FDCP-1 cells were separated in cytoplasmic, nuclear and mitochondrial fractions by differential centrifugation: cells were lysed by douncing in hopotonic lysis buffer contained 10 mM Hepes pH 7.3, 200 mM mannitol, 70 mM sucrose, 1 mM EGTA, 1 mM NaF, 0.1 mM NaₓVO_{4y} and protease inhibitor Complete ™ (Roche Molecular Biochemicals). Nuclear fractions were collected in low speed pellets (750 g), mitochondrial fractions in high speed pellets (15000 g) and cytoplasmic fractions are represented by the high speed supernatant. These fractions were subsequently subjected to western-blotting using the antiserum directed against the C-terminus of p12. The bulk of cellular p12 was found in the cytosol of cells grown in the presence of IL-3. After cytokine removal the total cellular p12-level increases as expected but now the majority of cellular p12 was found in the mitochondrial fraction (Fig. 4). The apparent absence of proteolytic processing of p12 suggests that it is not imported into mitochondria but rather binds to the outer mitochondrial membrane The quality of subcellular fractionations was controlled by the mitochondrial marker porin and the cytoplasmic localized caspase-2 (Fig. 4).

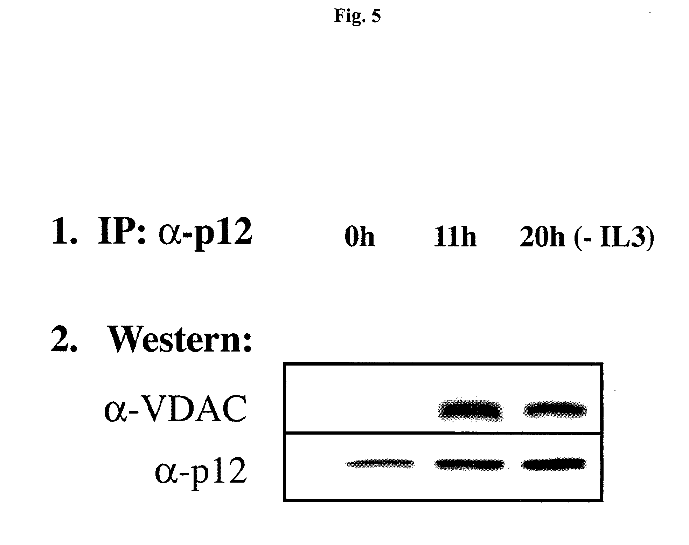

Next it was investigated whether the presence of p12 in the mitochondrial fraction in the absence of cytokine is mediated through interaction with components of the outer mitochondrial membrane. Potential candidates with a demonstrated role in regulation of cell viability were members of the Bcl-2 family and components of mitochondrial channels. For this purpose cell lysates were prepared from cells grown in the presence or absence of cytokine and p12 was immmunoprecipitated from the cell lysates (lysis buffer contained 20 mM Hepes pH7.5, 150 mM NaCl, 1 mM EDTA, 10% glycerol, 1 % TritonX-100, 1mM DTT, 1 mM NaF, 0.1 mM NaₓVO_{4y}) using antisera directed against the p12 C-terminus and protein A-sepharose beads (Pharmacia). The immunoprecipitates were subjected to western blotting and filters were probed with the following antisera: rabbit anti p12 antisera as described above, anti VDAC (31 HL, Calbiochem). Whereas no stable interactions of p12 with Bcl-2 family members was detectable (not shown), the outer mitochondrial membrane component VDAC specifically coprecipitated with p12 from cytokine starved cell-lysates but not from lysates of cells grown in the presence of cytokine (Fig. 5).

The voltage dependent anion channel VDAC has recently been implicated in other cell systems in the release of proapoptotic activities from mitochondria and is potentially regulated by Bcl-2 family members (Shimizu et al., 1999). This suggests that p12 plays an important role in a VDAC dependent release of proapoptotic activities in cytokine dependent myeloid cells.

### References

Beug, H., et al., 1995, Avian hematopoietic cell culture: in vitro model systems to study oncogenic transformation of hematopoietic cells. Methods Enzymol, 254:41-76.
Boulianne, G. L., et al., (1984), Nature 312: 643-646 Chao, D.T. and S.J. Korsmeyer, 1998, BCL-2 family: regulators of cell death. Annu Rev Immunol, 16: 395-419.
Coulie, P.G., 1997, Mol. Med. Today 3, 261-268 Delahunty, et al., 1996, Am. J. Hum. Genet. 58: 1239-1246.
Desagher, S. and Martinou, J.C., 2000, Mitochondria as the central control point of apoptosis. Trends Cell Biol, Sep. 10(9):369-77.
Fotin-Mleczek, M., et al., 2000, Detection of protein-protein interactions using a green fluorescent protein-based mammalian two-hybrid system [In Process Citation]. Biotechniques, Jul. 29(1):22-6.
Gabig, T.G., et al., 1994, Requiem: a novel zinc finger gene essential for apoptosis in myeloid cells. J Biol Chem, 269(47):29515-9.
Graziano, R.F., et al., (1995), J. Immunol. 155: 4996-5002
Jacobson, M.D., Weil, M., and Raff, M.C., 1997, Programmed cell death in animal development. Cell, 88(3):347-54.
Jarvik, J.W. and C.A. Telmer, 1998, Epitope tagging. Annu Rev Genet, 32:601-18.
Keown, W.A., Campbell, C.R., and Kucherlapati, R.S., 1990, Methods for introducing DNA into mammalian cells. Methods Enzymol, 185:527-37.
Köhler, G. and Milstein, C., 1975, Nature 265, 495-497 Neuberger, M.S., et al., 1984, Nature 312: 604-608 Nicholson, D.W. and Thornberry, N.A., 1997, Caspases: killer proteases. Trends Biochem Sci, 22(8): 299-306.
Paulus, W., et al., 1996, Self-contained, tetracycline-regulated retroviral vector system for gene delivery to mammalian cells. J Virol, 70(1):62-7.
Peng, S.S., Chen, C.Y., and Shyu, A.B., 1996, Functional characterization of a non-AUUUA AU-rich element from the c-jun proto-oncogene mRNA: evidence for a novel class of AU-rich elements. Mol Cell Biol, 16(4):1490-9.
Raff, M.C., 1996, Size control: the regulation of cell numbers in animal development. Cell, 86(2):173-5.
Raff, M., 1998, Cell suicide for beginners [news]. Nature, 396(6707):119-22.
Riechmann, L., et al., (1988), Nature 332: 323-327 Riley, et al., 1990, N.A.R. 18:2887-2890
Saiki, R.K., et al., 1985, Enzymatic amplification of beta-globin genomic sequences and restriction site analysis for diagnosis of sickle cell anemia. Science, 230(4732):1350-4.
Sambrook, et al. Molecular Cloning: A Laboratory Manual, CSH Press 1989, pp.14.2-14.33.
Shimizu, S., Narita, M., and Tsujimoto, Y., 1999, Bcl-2 family proteins regulate the release of apoptogenic cytochrome c by the mitochondrial channel VDAC [see comments]. Nature, 399(6735):483-7.
Wagner, R.W., et al., 1996, Nature Biotechnology 14: 840-844
Williams, G.T., et al., 1990, Haemopoietic colony stimulating factors promote cell survival by suppressing apoptosis. Nature, 343(6253):76-9.
Wilson, I.A., et al., 1984, The structure of an antigenic determinant in a protein. Cell, 37(3): 767-78.
Winnacker, 1987, From Genes to Clones, New York: VCH Publishers, pp. 451-481

## Claims

1. A murine polypeptide designated p12 with the amino acid sequence as set forth in SEQ ID NO:2 or a polypeptide encoded by a polynucleotide which hybridises under stringent conditions to a polynucleotide having a nucleotide sequence as set forth in SEQ ID NO:1.

2. An isolated DNA molecule comprising a polynucleotide with the nucleotide sequence as set forth in SEQ ID NO:1 encoding murine p12 polypeptide or an isolated DNA molecule comprising a polynucleotide which hybridises under stringent conditions to a polynucleotide having a nucleotide sequence as set forth in SEQ ID NO:1

3. A human polypeptide designated p12 with the amino acid sequence as set forth in SEQ ID NO:4 or a polypeptide encoded by a polynucleotide which hybridises under stringent conditions to a polynucleotide having a nucleotide sequence as set forth in SEQ ID NO:3.

4. An isolated DNA molecule comprising a polynucleotide with the nucleotide sequence as set forth in SEQ ID NO:3 encoding human p12 polypeptide or an isolated DNA molecule comprising a polynucleotide which hybridises under stringent conditions to a polynucleotide having a nucleotide sequence as set forth in SEQ ID NO:3.


SEQUENCE LISTING

<110> Boehringer Ingelheim International GmbH

<120> Pro-apoptotic proteins and DNA molecules encoding them

<130> case 14-061prio

<140>
<141>

<160> 4

<170> PatentIn Ver. 2.1

<210> 1
<211> 505
<212> DNA
<213> Mus musculus

<220>
<221> 5'UTR
<222> (1)..(47)

<220>
<221> CDS
<222> (48)..(389)

<220>
<221> 3'UTR
<222> (390)..(505)

<400> 1
   cacctcgccc gaggcagtga gggaccaggc tctccaagga cagaaaa atg gcg gcc 56
   Met Ala Ala
   1
   ctg ggg tcc ccg gcg cgc act ctg cgg ggc ctt ctg agg gag ctg cgc 104
   Leu Gly Ser Pro Ala Arg Thr Leu Arg Gly Leu Leu Arg Glu Leu Arg
   5 10 15
   tac ctg aac gcg gcc acc ggg cgg cca tat cgc gac aca gcg gcc tac 152
   Tyr Leu Asn Ala Ala Thr Gly Arg Pro Tyr Arg Asp Thr Ala Ala Tyr
   20 25 30 35
   cgg tac ctc gtt aag gct ttc cga gca cat cgg gtt acc agt gag aag 200
   Arg Tyr Leu Val Lys Ala Phe Arg Ala His Arg Val Thr Ser Glu Lys
   40 45 50
   ttg tgt aga gcc caa cac gaa ctt cac ttc caa gct gcc acc tat ctc 248
   Leu Cys Arg Ala Gln His Glu Leu His Phe Gln Ala Ala Thr Tyr Leu
   55 60 65
   tgc ctt ttg agt agc atc cgg caa cat gta gcc ctt cat cag gaa ttt 296
   Cys Leu Leu Ser Ser Ile Arg Gln His Val Ala Leu His Gln Glu Phe
   70 75 80
   cat ggc aag ggt gag cgt tca gtg gag gag tct gct ggt tta gtg ggc 344
   His Gly Lys Gly Glu Arg Ser Val Glu Glu Ser Ala Gly Leu Val Gly
   85 90 95
   ctc cag ttg ccc cgt cag cct gga ggg aag ggc tgg gag ccg tga       389
   Leu Gln Leu Pro Arg Gln Pro Gly Gly Lys Gly Trp Glu Pro
   100                 105                 110
   tgcagagagt cctcagatgt tccttcattc aagagtttaa ccatttctaa caatatgtag 449
   ttatcattaa atctttttta aagtgtaaaa aaaaaaaaaa aaaaaaaaaa aaaaaa     505

<210> 2
<211> 113
<212> PRT
<213> Mus musculus

<400> 2
Met Ala Ala Leu Gly Ser Pro Ala Arg Thr Leu Arg Gly Leu Leu Arg
1               5                   10                  15

Glu Leu Arg Tyr Leu Asn Ala Ala Thr Gly Arg Pro Tyr Arg Asp Thr
            20                  25                  30

Ala Ala Tyr Arg Tyr Leu Val Lys Ala Phe Arg Ala His Arg Val Thr
        35                  40                  45

Ser Glu Lys Leu Cys Arg Ala Gln His Glu Leu His Phe Gln Ala Ala
    50                  55                  60

Thr Tyr Leu Cys Leu Leu Ser Ser Ile Arg Gln His Val Ala Leu His
65                  70                  75                  80

Gln Glu Phe His Gly Lys Gly Glu Arg Ser Val Glu Glu Ser Ala Gly
                85                  90                  95

Leu Val Gly Leu Gln Leu Pro Arg Gln Pro Gly Gly Lys Gly Trp Glu
            100                 105                 110

Pro

<210> 3
<211> 467
<212> DNA
<213> Homo sapiens

<220>
<221> 5'UTR
<222> (1)..(42)

<220>
<221> CDS
<222> (43)..(384)

<220>
<221> 3'UTR
<222> (385)..(467)

<400> 3
tgcggccgcc gttgggcacc acgctcggag aaggacagga ca atg gcg gcc tta     54
                                               Met Ala Ala Leu
                                               1

ggg tcc ccg gcg cac act ttt cga gga ctt ctg cgg gag ttg cgc tac    102
Gly Ser Pro Ala His Thr Phe Arg Gly Leu Leu Arg Glu Leu Arg Tyr
  5                  10                  15                  20

ctg agc gcg gcc acc ggc cga ccc tat cgc gac acc gcg gcc tat cgg    150
Leu Ser Ala Ala Thr Gly Arg Pro Tyr Arg Asp Thr Ala Ala Tyr Arg
                 25                  30                  35

tac ctt gtg aag gct ttc cgt gca cat cgg gtc acc agt gaa aag ttg    198
Tyr Leu Val Lys Ala Phe Arg Ala His Arg Val Thr Ser Glu Lys Leu
             40                  45                  50

tgc aga gcc caa cat gag ctt cat ttc caa gct gcc acc tat ctc tgc    246
Cys Arg Ala Gln His Glu Leu His Phe Gln Ala Ala Thr Tyr Leu Cys
         55                  60                  65

ctc ctg cgt agc atc cgg aaa cat gtg gcc cta cat cag gaa ttt cat    294
Leu Leu Arg Ser Ile Arg Lys His Val Ala Leu His Gln Glu Phe His
     70                  75                  80

ggc aag ggt gag cgc tcg gtg gag gag tct gct ggc ttg gtg ggt ctc    342
Gly Lys Gly Glu Arg Ser Val Glu Glu Ser Ala Gly Leu Val Gly Leu
 85                  90                  95                 100

aag ttg ccc cat cag cct gga ggg aag ggc tgg gag cca tga            384
Lys Leu Pro His Gln Pro Gly Gly Lys Gly Trp Glu Pro
                105                 110

acatggagaa tatccttgga tgctgcattc ataggagaat tgaataattt ctatcaatat 444

gtatttatca ttaaattttt ttt                                          467



<210> 4
<211> 113
<212> PRT
<213> Homo sapiens

<400> 4
Met Ala Ala Leu Gly Ser Pro Ala His Thr Phe Arg Gly Leu Leu Arg
  1               5                  10                  15

Glu Leu Arg Tyr Leu Ser Ala Ala Thr Gly Arg Pro Tyr Arg Asp Thr
             20                  25                  30

Ala Ala Tyr Arg Tyr Leu Val Lys Ala Phe Arg Ala His Arg Val Thr
         35                  40                  45

Ser Glu Lys Leu Cys Arg Ala Gln His Glu Leu His Phe Gln Ala Ala
     50                  55                  60

Thr Tyr Leu Cys Leu Leu Arg Ser Ile Arg Lys His Val Ala Leu His
 65                  70                  75                  80

Gln Glu Phe His Gly Lys Gly Glu Arg Ser Val Glu Glu Ser Ala Gly
                 85                  90                  95

Leu Val Gly Leu Lys Leu Pro His Gln Pro Gly Gly Lys Gly Trp Glu
            100                 105                 110

Pro